# EUROPÄISCHE PATENTSCHRIFT

(11) **EP 1 392 856 B1**
(45) Veröffentlichungstag und Bekanntmachung des Hinweises auf die Patenterteilung: **14.01.2009**
(21) Anmeldenummer: 01991710.3
(22) Anmeldetag: 22.12.2001
(51) Int. Cl.: C12Q 1/68

(54) **VERFAHREN FÜR DIE SIMULTANE AMPLIFIKATION VIELER SEQUENZEN IN EINER PCR-REAKTION UND DEREN MARKIERUNG**
METHOD FOR THE SIMULTANEOUS AMPLIFICATION OF MULTIPLE SEQUENCES IN A PCR REACTION AND MARKING THEREOF
PROCEDE D'AMPLIFICATION SIMULTANEE DE PLUSIEURS SEQUENCES DANS UNE REACTION EN CHAINE DE LA POLYMERASE ET LEUR MARQUAGE

(30) Priorität: 22.12.2000 DE 10065814
(43) Veröffentlichungstag der Anmeldung: 03.03.2004
(73) Patentinhaber: Epigenomics AG, 10178 Berlin (DE)
(72) Erfinder: DISTLER, Jürgen, 10825 Berlin (DE); BERLIN, Kurt, 14532 Stahnsdorf (DE); OLEK, Alexander, 10115 Berlin (DE)
(74) Vertreter: Schubert, Klemens
(86) Internationale Anmeldenummer: PCT/DE2001/004951
(87) Internationale Veröffentlichungsnummer: WO 2002/052040

(56) Entgegenhaltungen:
- WO-A-01/07647
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048
- SCHUELKE M: "An economic method for the fluorescent labeling of PCR fragments." NATURE BIOTECHNOLOGY. UNITED STATES FEB 2000, Bd. 18, Nr. 2, Februar 2000 (2000-02), Seiten 233-234, XP002226508 ISSN: 1087-0156 in der Anmeldung erwähnt

## Beschreibung

### Gebiet der Erfindung

Die Erfindung bezieht sich auf die besonders rationelle Herstellung komplexer, markierter Amplifikate in einer PCR-Reaktion. Diese Amplifikate können nachfolgend hinsichtlich ihrer Sequenz mittels verschiedenster Methoden untersucht werden. Besonders ist das Verfahren zur Analyse von Cytosin-Methylierungsmustern in DNA-Proben geeignet.

### Stand der Technik

Die Polymerasekettenreaktion (PCR) ist eine Methode, mit der im Prinzip jede DNA selektiv amplifiziert werden kann. Diese Methode beinhaltet den Gebrauch eines Satzes von meist zwei Oligonukleotiden mit vorbestimmter Sequenz, den sogenannten Primern, die an zu ihnen komplementäre DNA Stränge hybridisieren und die Grenzen für die zu amplifizierende Sequenz definieren.

Die Oligonukleotide initiieren die DNA Synthese, die durch eine thermostabile DNA Polymerase katalysiert wird. Jede Runde der Synthese wird typischerweise durch einen Schmelz- und Reannelierungsschritt getrennt. Dies erlaubt es, eine gegebene DNA Sequenz mehrere hundert Male in weniger als einer Stunde zu amplifizieren.

Durch die Einfachheit und Reproduzierbarkeit dieser Reaktionen hat die PCR eine weite Akzeptanz erreicht. Zum Beispiel wird die PCR für die Diagnose ererbter Fehlfunktionen und bei Verdacht auf Erkrankungen verwendet.

Oft wird jedoch auch eine Amplifikation einer gegebenen Probe durchgeführt, einfach um das Material für eine nachfolgende Untersuchung zu vermehren. Die zu untersuchende Probe wird in diesem Fall zunächst amplifiziert, entweder ausgehend von genomischer DNA, oder z. B. isolierter mRNA. Meist ist es erforderlich, mindestens einen der Primer zu markieren, z. B. mit einem Fluorenzenzfarbstoff, um das Fragment in nachfolgenden Experimenten identifizieren zu können.

Eine besonders einfache und kostengünstige Variante zum Anbringen einer Markierung an die Amplifikate stellt die Methode dar, Primer zu verwenden, die zwei Domänen haben. Die eine hybridisiert spezifisch an die zu amplifizierende Region, während die andere nur die Funktion hat, mit einem markierten Oligonukleotid zu hybridisieren. Dieses Oligonukleotid kann für verschiedenste Amplifikationen immer das gleiche sein, wenn immer die gleiche für die Markierung zuständige Domäne des Primers verwendet wird. Insbesondere bei teuren Markierungen ist dies eine kostengünstige Lösung. Beispielsweise werden für diese Reaktion drei verschiedene Oligonukleotide eingesetzt, ein sequenzspezifischer Vorwärtsprimer mit M13(-21) Schwanz, ein sequenzspezifischer reverser Primer und ein universelles fluoreszenzmarkiertes M13(-21) Oligonukleotid (Schuelke et al., An economic method for the fluorescent labeling of PCR fragments 2:18, 2000), welches an den M13(-21) Schwanz des Vorwärtsprimers bindet.

Diese amplifizierte DNA wird für die Identifikation von Mutationen und Polymorphismen benutzt. Als analytische Methode dafür kommt z.B. die Primer Extension Reaktion, Sequenzierung nach Sanger oder z. B. Restriktionsverdaus und nachfolgende Untersuchung auf z. B. Agarosegelen in Frage.

Zur Untersuchung der DNA wird über die Untersuchung der Basensequenz hinaus häufig das Verhältnis von den DNA Basen Cytosin zu 5-Methylcytosin herangezogen, bzw. es werden einzelne Cytosinpositionen auf Methylierung hin untersucht.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek A, Oswald J, Walter J. A modified and improved method for bisulphite based cytosine methylation analysis. Nucleic Acids Res. 1996 Dec 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifziert und entweder komplett sequenziert (Olek A, Walter J. The pre-implantation ontogeny of the H19 methylation imprint. Nat Genet. 1997 Nov;17(3):275-6) oder einzelne CytosinPositionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun 15;25(12):2532-4) nachgewiesen. Zudem ist auch der Nachweis durch Hybridisierung beschrieben worden (Olek et al., WO 99 28498).

Weitere Publikationen, die sich mit der Anwendung der Bisulfit-Technik zum Methylierungsnachweis bei einzelnen Genen befassen, sind:

Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97 46705, WO 95 15373 und WO 45560.

Zur Analyse der PCR-Produkte müssen diese z.B. mit einer Fluoreszenz-Markierung oder radioaktiven Markierung versehen werden. Diese Markierungen können entweder an den Primern oder an den Nukleotiden angebracht werden. Besonders geeignet für Fluoreszenzmarkierungen ist das einfache Anbringen von Cy3 und Cy5 Farbstoffen am 5'-Ende der jeweiligen Primer. Ferner kommen als Fluoreszenzfarbstoffe 6-Carboxyfluoreszin (FAM), Hexachlor-6-carboxyfluoreszin (HEX), 6-Carboxy-x-rhodamin (ROX) oder Tetrachlor-6-carboxyfluoreszin (TET) in Frage. Diese Farbstoffe sind jedoch vergleichsweise teuer.

Für die Analyse von Genen, wo das mutierte Allel oder der Polymorphismus gut charakterisiert sind, ist die Amplifikation von einzelnen definierten Regionen der DNA manchmal ausreichend. Wenn man jedoch undefinierte Gene analysiert, sind meistens eine Vielzahl von PCR Reaktionen notwendig, um kritische Deletionen oder Änderungen von Basen zu identifizieren. Noch komplizierter ist die Etablierung einer Multiplex-PCR, bei der ein Forwardprimer und eine Vielzahl von Reversprimern verwendet werden, um definierte Genabschnitte zu amplifizieren.

Während die Annelierungstemperatur und die Primerkonzentration bis zu einem gewissen Grad berechnet werden können, müssen die Bedingungen für jede einzelne Multiplexreaktion im allgemeinen experimentell bestimmt werden. Da die Wahrscheinlichkeit einer nichtspezifischen Initialreaktion mit jedem zusätzlichen Primer ansteigt, müssen die Bedingungen bei weiterer Primerzugabe meistens modifiziert werden. Des weiteren vermehren sich Artefakte, die durch die Konkurrenz um Resourcen entstehen, z.B. um die Primer bei der Multiplex-PCR. Dies liegt daran, dass die Ausbeuten von ungleich amplifizierten Fragmenten mit jedem Zyklus ansteigen.

Durch die angesprochenen Schwierigkeiten kann die Entwicklung eines neuen diagnostischen Tests sehr arbeits- und kostenintensiv sein.

Weighardt et al. (PCR Methods and App. 3:77, 1993) beschreiben den Gebrauch von 5'-verlängerten Oligonukleotiden für die PCR. Diese Amplifikationsmethode beinhaltet als charakteristisches Merkmal die separate Annelierung und Primer Verlängerungsreaktion für jeden individuellen Primer, was in einem Multiplex-Kontext nicht durchführbar ist.

Wie gezeigt, ist es gegenwärtig Stand der Technik, zur Identifizierung von Cytosin-Methylierung die Proben-DNA mit Bisulfit zu behandeln und nachfolgend einfache Amplifikationen durchzuführen. Es fehlt jedoch ein Verfahren, dass es erlaubt, unter Verwendung von Primer mit zwei Domänen eine besonders spezifische zwei-Stufen-Amplifikation durchzuführen, die besonders spezifisch eine Vielzahl von Fragmenten gleichzeitig bereitstellt und die zugleich das Problem löst, dass normalerweise eine Vielzahl von markierten und entsprechend teuren Primern für eine solche komplexe Amplifikation eingesetzt werden muss.

### Aufgabenstellung

Es soll ein Verfahren bereitgestellt werden, welches die Nachteile des Standes der Technik überwindet. Es soll zum einen eine sehr spezifische 2 Stufen PCR mit hoher Multiplexierbarkeit bereitstellen, die zugleich das Problem der kostenintensiven Markierungen löst. Das Verfahren soll sich insbesondere zum Nachweis von Cytosinmethylierung in DNA-Proben eignen.

### Beschreibung

Beschrieben wird ein Verfahren zur Amplifikation von Nukleinsäuren.

Die Nukleinsäuren werden besonders bevorzugt aus einer genomischen DNA-Probe erhalten, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Die Nukleinsäureprobe wird zu Beginn besonders bevorzugt in Agarose eingebettet und mit einer Bisufitlösung (= Disulfit, Hydrogensulfit) umgesetzt, wobei 5-Methylcytosin unverändert bleibt und Cytosin in Uracil oder eine andere im Basenpaarungsverhalten dem Uracil ähnliche Base umgewandelt. Bei dieser chemischen Behandlung ist ein die DNA-Duplex denaturierendes Reagenz und/oder ein Radikalfänger vorhanden.

Da im humanen Genom lediglich ca. 3 % der Cytosinbasen methyliert vorliegen, enthält Bisulfit-behandelte DNA zum größten Teil die Basen A, G, T und U; das heißt bezüglich ihres Basenpaarungsverhaltens lediglich 3 Basen (A, G und T), da U und T das gleiche Paarungsverhalten zeigen.

Dann werden die zu amplifizierenden Abschnitte mit mindestens zwei Primeroligonukleotiden hybridisiert, welche zwei Domänen aufweisen, von denen die am 3'-Ende befindliche, sequenzspezifische Domäne an den zu amplifizierenden Abschnitt hybridisiert, während die am 5'-Ende befindliche, generische Domäne nicht hybridisiert. Im nächsten Schritt wird eine erste Amplifikationsreaktion mittels einer Polymerase durchgeführt. In einem weiteren Schritt wird an die Amplifikate ein markiertes Primeroligonukleotid hybridisiert, welches an die generische Domäne der ersten Primer hybridisiert. Bevorzugt wird darauf eine zweite Amplifikation mit einer Polymerasereaktion durchgeführt.

Für die Amplifikation wird besonders bevorzugt eine hitzebeständige DNA-Polymerase verwendet.

Wird Bisulfit-behandelte DNA amplifiziert, führt dies dann zu DNA-Fragmenten, die sich dadurch auszeichnen, dass der (+)-Strang die Basenzusammensetzung A, T und G, sein komplementärer (-)-Strang die Zusammensetzung A, T und C besitzt. Daraus ergibt sich, dass im (+)- oder (-)-Strang nie oder selten die C und G Nukleobasen gleichzeitig vorhanden sind. Durch diese Eigenschaft von Bisufitbehandelter DNA und seinen Amplifikationsprodukten ist es möglich Primer herzustellen, die mit Bisulfit Templat-DNA keine unspezifischen PCR-Produkte ergeben und so als generell anwendbare Markierungsoligonukleotide bzw. Detektionssonden für PCR-Amplifikate von Bisulfit-behandelter DNA verwendet werden können.

Voraussetzung hierfür ist, dass für die Amplifikation von Bisulfit-behandelter DNA genspezifische Primer des Typs 1 und 2 entworfen werden, die neben einer Gen-spezifischen Domäne eine generische Domäne besitzen. Diese generische Primerdomäne ermöglicht die Markierung der DNA-Fragmente mit den generell anwendbaren Markierungsoligonukleotiden (Typ M1 und Typ M2) bzw. ihre Detektion mit den generellen Markierungsoligonukleotiden (Typ M1 und Typ M2) durch Hybridisierungsverfahren. Für die Amplifikation spezifischer Gene oder mehrerer Gene in einer Multiplex-PCR sind mindestens 2 Primer notwendig, ein Primer des Typs 1 und der zweite vom Typ 2 (siehe unten). Mit diesen Primern werden die entsprechenden DNA-Fragmente amplifiziert.

### Aufbau und Eigenschaften der Primer

Primer Typ1:

generische Domäne                    genspezifische Domäne
5´-----------------------------------------------------------3´
enthält A,T,C,G                      enthält A, T, G

### Primer Typ2:

generische Domäne                    genspezifische Domäne
5´-----------------------------------------------------------3´
enthält A,T,C,G                      enthält A, T, C

### Primer TypM1:

Sequenz identisch mit generischer Domäne des Typ1 Primers
Mod-5´------------------------3´
enthält A,T,C,G, Mod: Modifikation oder Markierung

### Primer TypM2:

Sequenz identisch mit komplementärer Sequenz der generischen Domäne des Typ2 Primers

Mod-5´--------------------------3´
enthält A,T,C,G, Mod: Modifikation oder Markierung

Bevorzugt enthält die generische Domäne der Primer des Typs 1 und 2 eine Sequenz, die aus A, C, G und T zusammengesetzt ist.

Die genspezifischen Domänen sind, wie der Name schon sagt, spezifisch für ein oder mehrere Genfragment(e). Im Fall der sequenzspezifischen Primer vom Typ 1 ist die Sequenz aus A, T und G Basen, im Fall der sequenzspezifischen Primer des Typs 2 aus A, T und C Basen zusammengesetzt.

In einer zweiten PCR-Reaktion mit einem generischen Primerpaar des Typs M1 und M2 werden diese Gene oder eine beliebige Anzahl von Genen, die aus PCR-Reaktionen mit Primerpaaren (Typ 1 und Typ 2)hervorgegangen sind, gleichzeitig reamplifiziert, wobei alle Primer des Typs 1 und die Primer des Typs 2 identische generische Dömänen besitzen (Beispiel 2 und 3). Die generischen Domänen der Primer des Typs 1 und 2 sind so entworfen, das die entsprechenden generischen Markierungsprimer (Typ M1 und Typ M2) mit der verwendeten Bisulfit Templat-DNA möglichst keine unspezifischen PCR-Produkte in eine PCR-Reaktion erzeugen. Die PCR-Reaktionen können sequentiell oder durch die geeignete Auswahl der Gen-spezifischen und generischen Primer, sowie die Durchführung der PCR, simultan in einer sogenannten Eintopf-Reaktion durchgeführt werden .

Generische Primer des Typs M1 und M2 können auch für die Detektion von PCR-Amplifikaten, die mit Primern des Typs 1 und Typs 2 erzeugt und z.B. auf DNA-Arrays, Nitrocellulose-, PVDF-Membranen oder anderen festen Oberflächen immobilisiert wurden, durch Hybridisierungsverfahren verwendet werden.

Die amplifizierten Abschnitte, die aus PCR-Fragmenten mit zwei Domänen aufweisenden Primern hervorgegangen sind, können auf Festphasen immobilisert werden. Dies geschieht durch chemische Reaktionen (z. B. durch die Einführung einer 5'-Aminofunktion) oder durch Hybridisierung an andere auf die Festphase immobiliserte Oligonukleotide.Die Amplifikate können dabei die durch die markierten generischen Primer des Typs M1/M2 detektiert werden. Bevorzugt sind die an den Amplifikaten angebrachten Markierungen an jeder Position der Festphase, an der sich eine Oligonukleotidsequenz befindet, identifizierbar.

Werden als generische Primer keine DNA-Oligomere jedoch PNA-Oligomere des Typs M1 und M22 verwendet, können PCR-Amplifikaten, die mit Primern des Typs 1 und Typs 2 erzeugt wurden, auch im MALDI-TOF identifiziert und quantifiziert werden.

Bei den Markierungen der Oligonukleotidprimer handelt es sich vorzugsweise um Fluoreszenzfarbstoffe mit unterschiedlichem Emissionsspektrum (z.B. Cy3, Cy5, FAM, HEX, TET oder ROX) oder um Fluoreszenzfarbstoff-Kombinationen im Falle von Energietranfer-Fluoreszenzfarbstoff markierten Primern. Die Markierungen können bevorzugt Radionuklide oder bevorzugt ablösbare Massenmarkierungen sein, die in einem Massenspektrometer nachgewiesen werden. Vorzugsweise können auch zur Markierung Moleküle verwendet werden, die erst in einer weiteren chemischen Reaktion ein Signal erzeugen. Bevorzugt sind die zur Markierung verwendeten Moleküle an definierten Stellen an eine Festphase gebunden, um über eine Festphasen-PCR die PCR-Produkte zu immobilisieren, die aus einer PCR-Reaktion und Domäneprimern hervorgegangen sind.

Die PCR-Fragmente sind vorzugsweise auf der Festphase in Form eines rechtwinkligen oder hexagonalen Gitters angeordnet.

Abschließend wird das Amplifikat hinsichtlich seiner Sequenz untersucht.

Die beschriebene DNA-Modifizierung erfolgt durch den Einsatz von identischen Primerpaaren mit einer gleichen fragmentspezifischen Modifizierung, das heißt das alle Markierungen identisch sind und die gleiche spezifische Reaktion ergeben. Durch diese Vereinfachungen können beträchtliche Kosten eingespart werden, da insbesondere die Farbstoff- oder Massemakierungen große Kosten verursachen.

Das Verfahren wird vorzugsweise verwendet zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Das Verfahren wird besonders bevorzugt verwendet zur Unterscheidung von Zelltypen oder Geweben oder zur Untersuchung der Zelldifferenzierung.

Die nachfolgenden Beispiele erläutern die Erfindung:

### Beispiel 1: Design und Aufbau genspezifischer und modularer Primer

Von den Genen OAT (ACCESSION ep30056) und MDR1 (ACCESSION X58723) wurden jeweils die nach einer Bisulfitbehandlung vorliegenden Sequenzen ermittelt. Basierend auf diesen chemisch vorbehandelten DNA-Sequenzen (siehe Anhang) wurden die folgenden genspezifischen Primer-Domänen hergestellt und mit kommerziell erhältlicher Analysesoftware untersucht, um die selbstkomplementären bzw. interkomplementären Sequenzbereiche in den Primersequenzen auszuschließen (siehe Tab 1 und 2). Diese Sequenzen wurden in nicht-modulare PCR-Primer OAT-fp, OAT-rp, MDR-fp und MDR-rp umgesetzt und in PCR-Reaktionen auf ihre Funktion getestet. Die Primerkombinationen für OAT und MDR1 lieferten bei ihrer Verwendung in PCR-Reaktionen (vgl. Beispiel 2) mit Bisulfit DNA, hergestellt nach publizierter Methode (Olek et al., Nucl. Acids. Res. 1996, 24, 5064-5066), die erwarteten Produkte von 479 nt und 633 nt (siehe Tab 1). Als Sequenz für die generischen PrimerDomäne wurden gen1-f, entsprechend der Sequenz des M13 universal Sequenzierprimers und gen2-f (Tab 2), sowie gen-r entsprechend des reversen M13 Sequenzierprimers ausgewählt. Die entsprechenden, auf diesen Sequenzen basierenden nicht-modularen Primer, zeigten in PCR-Reaktion auf Bisulfit DNA, unter verschiedenen Reaktionsbedingungen, in den Primerpaarkombinationen gen1-fp, gen-rp, sowie gen2-fp, gen-rp, keine erkennbare PCR-Produkte.

Die modularen Primer zur Amplifikation von OAT und MDR1-Genbereichen ergeben sich durch die Fusion der generischen Sequenzen mit den entsprechenden genspezifischen Domänen, um die modularen Primer, OAT-f1mp, OAT-f2mp, OAT-rmp, MDR-f1mp, MDR-f2mp und MDR-rmp zu erhalten (Tab 3).

Tab 1. Genspezifische und generische Primerdomäne

| Gen | Vorwärtsprimer | Reversprimer | PCR-Fragment |
|---|---|---|---|
| OAT | OAT-f | OAT-r | 479 nt |
| MDR1 | MDR-f | MDR-r | 633 nt |
| | | | |
| generisch | gen1-f | gen-r | |
| | gen2-f | | |

Tab 2. Nicht-modulare Primersequenzen

| | |
|---|---|
| OAT-fp | TTTGGAGGTGGATTTAGAGGTATAATTAA |
| OAT-rp | AAACRTCACTACAACTTAAAAACTAA |
| | |
| MDR-fp | TAAGTATGTTGAAGAAAGATTATTGTAG |
| MDR-rp | TAAAAACTATCCCATAATAACTCCCAAC |
| | |
| gen1-fp | GTAAAACGACGGCCAGT |
| gen2-rp | GTAAAACCAGGGCCAGT |
| gen-rp | CAGGAAACAGCTATGAC |
| | |
| gen1-fp5 | Cy5-GTAAAACGACGGCCAGT |
| gen2-rp5 | Cy5-GTAAAACCAGGGCCAGT |
| gen-rp5 | Cy5-CAGGAAACAGCTATGAC |

Tab 3. Modulare Primer

| Name | Sequenz |
|---|---|
| | generische Domäne - genspezifische Domäne |
| OAT-f1mp | GTAAAACGACGGCCAGT-TTGGAGGTGGATTTAGAGGTATAATTAA |
| OAT-f2mp | GTAAAACCAGGGCCAGT-TTGGAGGTGGATTTAGAGGTATAATTAA |
| OAT-rmp | CAGGAAACAGCTATGAC-AAACRTCACTACAACTTAAAAACTAA |
| | |
| MDR-f1mp | GTAAAACGACGGCCAGT-TAAGTATGTTGAAGAAAGATTATTGTAG |
| MDR-f2mp | GTAAAACCAGGGCCAGT-TAAGTATGTTGAAGAAAGATTATTGTAG |
| MDR-rmp | CAGGAAACAGCTATGAC-TAAAAACTATCCCATAATAACTCCCAAC |

### Beispiel 2: Gen-spezifische Amplifikation von OAT und MDR1 mit modularen Primern

Die Amplifikation von OAT- und MDR1-Genbereichen erfolgte mit Qiagen Hotstart-Polymerase entsprechend der Herstellerangaben (Qiagen, Hilden) in einem Reaktionsvolumen von 20 µl.

### Reaktionsansatz (allgemein):

| | |
|---|---|
| Bisulphit-DNA (10 ng) | 1 µl |
| Reaktions-Puffer 10x (Qiagen, Hilden) | 2 µl |
| dNTP-Mix (10mM each) | 2 µl |
| Primer1 6,25 pmol | 2 µl |
| Primer2 6,25 pmol | 2 µl |
| Polymerase (Qiagen, Hilden) (0,5 U) | 0,5 µl |
| Wasser | 11,5 µl |

Die PCR-Reaktion wurde im Master Cycler Gradient (Eppendorf, Hamburg) mit folgendem Programm durchgeführt.

### Programm:

15 min 96 °C 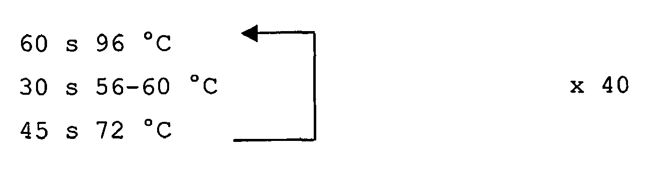 10 min 72 °C

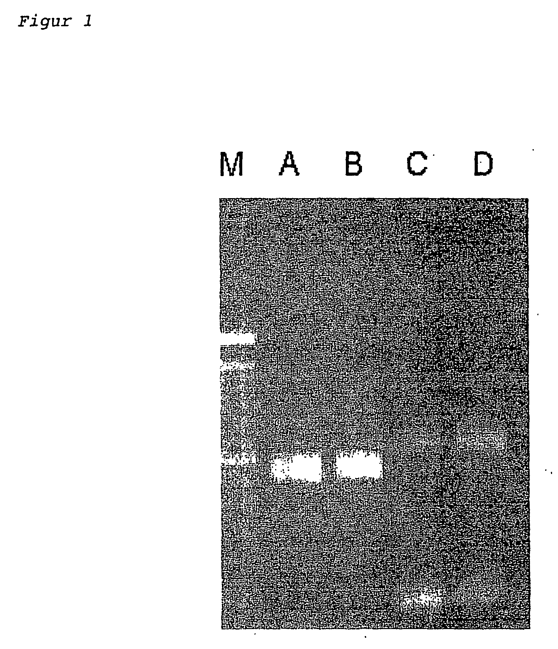

In Tab 4 sind die verwendeten Primerkombinationen und Annealing-Temperaturen für die Amplifikation von OAT und MDR1 zusammengefaßt. Die erzeugten PCR-Amplifikate wurden durch Agarosegel-Elektrophorese (1,5 % Agarose in 0,5xTBE-Puffer, Manniatis et al.) analysiert. Hierfür wurden 4 µl des PCR-Ansatzes der Gelelektrophorese unterzogen, das Ergebnis ist in Abb 1 dargestellt.

**Tab 4 PCR-Primerkombinationen**

| Gen | Primer 1 | Primer 2 | Annealing Tmp. | Spur in Abb 1 |
|---|---|---|---|---|
| | | | | |
| OAT | OAT-f1mp | OAT-rmp | 55,6 °C | A |
| OAT | OAT-f2mp | OAT-rmp | 55,6 °C | B |
| MDR1 | MDR-f1mp | MDR-rmp | 59 °C | C |
| MDR1 | MDR-f2mp | MDR-rmp | 59 °C | D |

### Beispiel 3: ReaMplifikation von OAT und MDR1 mit generischen Primern

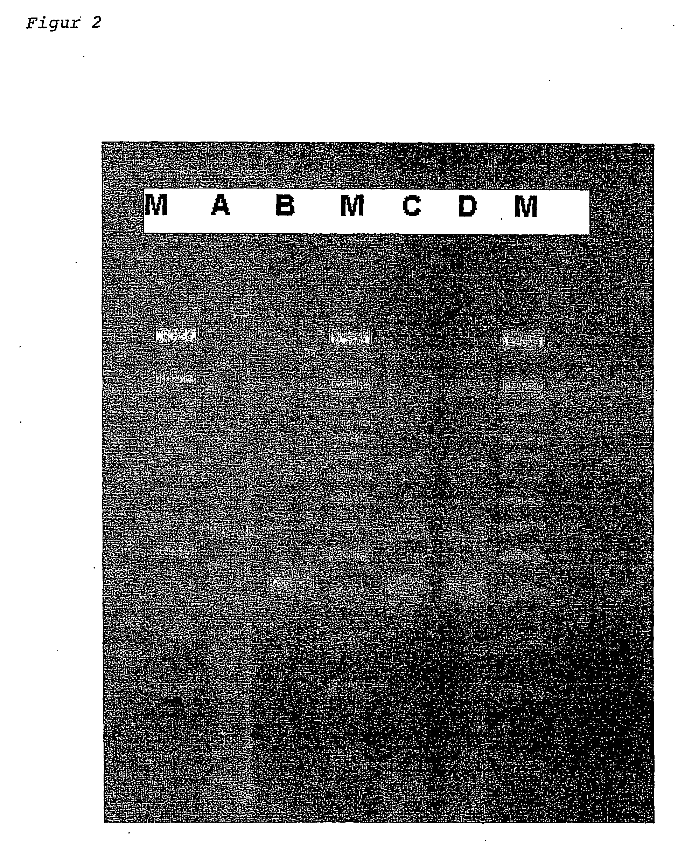

Für die Reamplifikation (= Markierungsamplifikation) von OAT- und MDR1-Genbereichen erfolgte mit Qiagen Hotstart-Polymerase entsprechend der Herstellerangaben (Qiagen, Hilden) in einem Reaktionsvolumen von 20 µl. .Hierbei wurden die PCR-Produkte aus der Gen-spezifischen PCR (siehe Beispiel 2, Abb 1) 1:1000 verdünnt und getrennt oder gemeinsam in einer PCR-Reaktion amplifiziert. Als Primer wurden die am 3'-Ende mit dem Floureszensfarbstoff, Cy5, modifizierte Primerpaare gen1-fp5 und gen-rp5, sowie gen2-fp5 und gen-rp5 verwendet. Die Ergebnisse dieser Markierungsamplifikation sind in Abb. 2 dargestellt.

### Reaktionsansatz (allgemein):

| | |
|---|---|
| Templat-DNA | 1 µl |
| Reaktionspuffer 10x (Qiagen, Hilden) | 2 µl |
| dNTP-Mix (10mM each) | 2 µl |
| Primerpaar (6,25 pmol je Primer) | 4 µl |
| Polymerase (Qiagen, Hilden) (0,5 U) | 0,5 µl |
| Wasser | 11,5 µl |

Die PCR-Reaktion wurde im Master Cycler Gradient (Eppendorf, Hamburg) mit folgendem Program durchgeführt:
15 min 96 °C 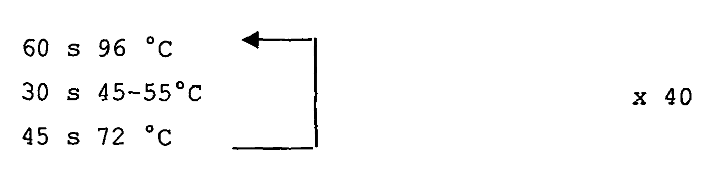 10 min 72 °C

Die erzeugten PCR-Amplifikate wurden durch Agarosegel-Elektrophorese (1,5 % Agarose in 0,5xTBE-Puffer, Manniatis et al.) analysiert. Hierfür wurden 4 µl des PCR-Ansatzes der Gelelektrophorese unterzogen, das Ergebnis ist in Abb 2 dargestellt. Unter den angegeben Bedinungen konnten sowohl OAT und MDR1, mit beiden generischen Primerpaaren erfolgreich amplifiziert und somit in einer PCR-Reaktion gleichzeit, identisch modifiziert werden.

### Beispiel 4: Reamplifikation mit einem Domänenprimer

Von ESR1 wurde die Bisulphit Sequenz erstellt. Basierend auf dieser DNA-Sequenz (siehe nachfolgend ESR1-PCR-Produkt) wurden folgende genspezifischen Primer-Domänen hergestellt und mit kommerziell erhältlicher Analysesoftware untersucht, um die selbstkomplementären bzw. interkomplementären Sequenzbereiche in den Primersequenzen auszuschließen (siehe Tab 5). Diese Sequenzen wurden in modulare PCR-Primer ER1-B-U-M13a, ER1-B-L-M13b, ER1-B-UM13b, ER1-B-L-M13a sowie die nicht-modularen PCR-Primer ER1-B-U, ER1-B-L umgesetzt und in PCR-Reaktionen auf ihre Funktion getestet. Die modularen Primer zur Amplifikation von ESR1-Genbereichen ergeben sich durch die Fusion der generischen Sequenzen M13a und M13b mit den genspezifischen Domänen ER1-B-U und ER1-B-L (Tab 5). Die Primerkombinationen ER1-B-L-M13b/ ER1-B-L-M13a, ER1-B-U-M13a/ ER1-B-L-M13a, ER1-B-L-M13b/ ER1-B-U-M13b und ER1-B-U/ER1-B-L lieferten bei ihrer Verwendung in PCR-Reaktionen (vgl.

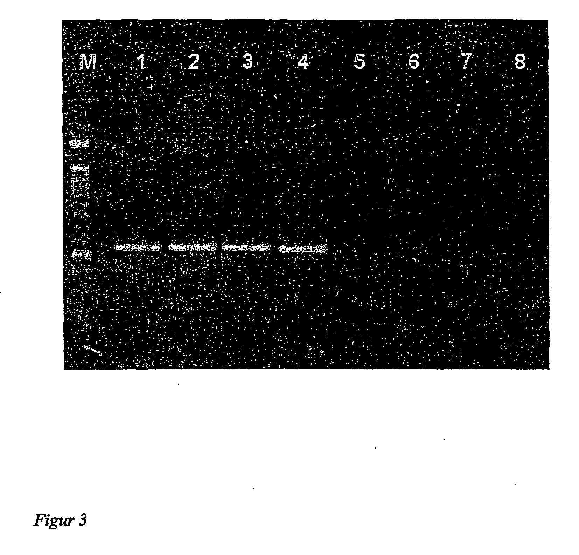

Fig. 3, Spur 1-4) mit bisulphitbehandelter DNA die erwarteten Produkte von 699 nt (modulare Primer) und 665 nt (nicht-modulare Primer). Hingegen wurden keine PCR-Produkte erhalten, wenn chemisch unbehandelte DNA in die PCR-Reaktion eingesetzt wurde (vgl. Fig. 3, Spur 5-8). Der PCR-Reaktionsansatz hatte folgende Zusammensetzung:

| | |
|---|---|
| DNA (10 ng) | 1 µl |
| Reaktions-Puffer 10x (Qiagen, Hilden) | 2,5 µl |
| dNTP-Mix (jeweils 25 mM) | 0,2 µl |
| Primer1 12,5 pmol | 1 µl |
| Primer2 12,5 pmol | 1 µl |
| Polymerase (Qiagen, Hilden) (0,5 U) | 0,2 µl |
| Wasser | 19,1 µl |

und wurde mit folgendem "Cycling"-Programm durchgeführt: 15 min 96 °C; 60 s 96 °C (x 40); 45 s 55 °C (x 40); 75 s 72 °C (x 40); 10 min 72 °C

**Tabelle 5: Gen-spezifische, molulare und generische Primer für die Amplifikation von ESR1**

| | Name | Sequenz |
|---|---|---|
| A | ER1-B-U-M13a | GTAAAACGACGGCCAGT*AGGAGGGGGAATTAAATAGA* |
| B | ER1-B-L-M13b | CAGGAAACAGCTATGAC*ACAATAAAACCATCCCAAATAC* |
| C | ER1-B-U-M13b | CAGGAAACAGCTATGAC*AGGAGGGGGAATTAAATAGA* |
| D | ERI-B-L-M13a | GTAAAACGACGGCCAGT*ACAATAAAACCATCCCAAATAC* |
| E | ER1-B-U | AGGAGGGGGAATTAAATAGA |
| F | ER1-B-L | ACAATAAAACCATCCCAAATAC |
| G | M13a | GTAAAACGACGGCCAGT |
| H | M13b | CAGGAAACAGCTATGAC |

Die mit den Primerkombinationen ER1-B-L-M13b/ ER1-B-L-M13a, ER1-B-U-M13a/ ER1-B-L-M13a, ER1-B-L-M13b/ ER1-B-UM13b und ER1-B-U/ER1-B-L (vgl. Fig. 3) erzeugten PCR-Produkte wurden mit Qiagen Hotstart-Polymerase entsprechend der Herstellerangaben (Qiagen, Hilden) in einem Reaktionsvolumen von 25 µl mit dem Primer M13a oder M13b oder einer Mischung dieser Primer reamplifiziert. Hierbei wurden die PCR-Produkte aus der PCR-Reaktion (siehe Fig. 3) 1:1000 verdünnt und in folgenden PCR-Reaktionen amplifiziert.
Reaktionsansatz (allgemein):
bei der Verwendung von einem Primer

| | |
|---|---|
| DNA (1:1000 verdünntes PCR-Fragment) | 1 µl |
| Reaktions-Puffer 10x (Qiagen, Hilden) | 2,5 µl |
| dNTP-Mix (jeweils 25 mM ) | 0,2 µl |
| Primer 12,5 pmol/µl | 2 µl |
| Polymerase (Qiagen, Hilden) (0,5 U) | 0,2 µl |
| Wasser | 19,1 µl |

bei der Verwendung eines Primerpaars

| | |
|---|---|
| DNA (1:1000 verdünntes PCR-Fragment) | 1 µl |
| Reaktion-Puffer 10x (Qiagen, Hilden) | 2,5 µl |
| dNTP-Mix (jeweils 25mM) | 0,2 µl |
| Primer1 12,5 pmol/µl | 2 µl |
| Primer2 12,5 pmol/µl | 1 µl |
| Polymerase (Qiagen, Hilden) (0,5 U) | 0,2 µl |
| Wasser | 19,1 µl |

Die PCR-Reaktion wurde im Master Cycler Gradient (Eppendorf, Hamburg) mit folgendem Programm durchgeführt: 15 min 96 °C; 60 s 96 °C (x 40); 45 s 55 °C (x 40); 75 s 72 °C (x 40); 10 min 72 °C

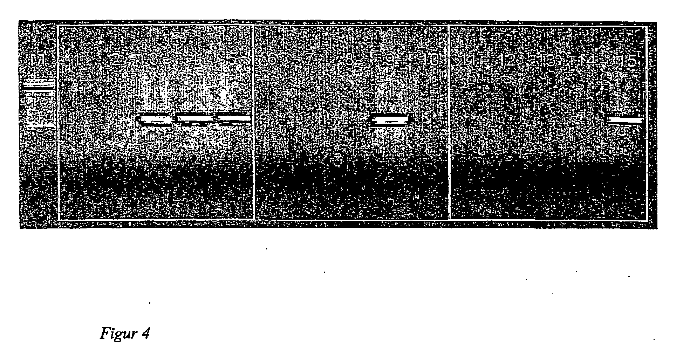

Die Reamplifikationen lieferten die erwarteten Ergebnisse (siehe Fig. 4). Mit der Mischung der generischen Primer M13a/M13b konnten die PCR-Produkte der Primerkombinationen ER1-B-L-M13b/ ER1-B-L-M13a, ER1-B-U-M13a/ ER1-B-L-M13a und ER1-B-L-M13b/ ER1-B-U-M13b reamplifiziert werden. Die Reamplifikationen der PCR-Produkte der Primerkombinationen ER1-B-U-M13a/ ER1-B-L-M13a konnten auch mit dem Primer M13a, die der Primerkombinationen ER1-B-L-M13b/ ER1-B-U-M13b mit dem Primer M13b, durchgeführt werden. Das PCR-Produkt, erhalten mit den nicht-modularen Primerkombinationen ER1-B-U/ER1-B-L, konnte erwartungsgemäß mit keinem generischen Primer oder einer Kombination der Primer M13a/M13b reamplifiziert werden (siehe Fig. 4: Analyse der ESR1 PCR-Produkte in einem 1,5 %igen Agarosegel (Manniatis et al.).

Figur 3: Jeweils 4 µl der PCR-Reaktionen der Primerkombinationen ER1-B-L-M13b/ ER1-B-L-M13a (Spur 1,5), ER1-B-UM13a/ ER1-B-L-M13a (Spur 2,6), ER1-B-L-M13b/ ER1-B-U-M13b (Spur 3,7) und ER1-B-U/ER1-B-L (Spur 4,8) auf Bisulphit behandelter DNA (Spur 1-4) und unbehandelter genomischer DNA (Spur 5-8) wurden analysiert. Spur M ist ein DNA-Fragment-Größenstandard.

ESR1-PCR-Produkt

AGGAGGGGGAATTAAATAGAAAGAGAGATAAATAGAGATATATCGGAGTTTGGTACG
GGGTATATAAGGTAGTATATTAGAGAAAGTCGGTTTTTGGATTCGTTTTTCGCGTTT
ATTTTAAGTTTAGTTTTTTTGGGTTATTTTTAGTAGATTTTCGTGCGTTTTCGTTT
TTTGGTCGTGAAATTTAGTTTTTATTTAGTAGCGACGATAAGTAAAGTAAAGTTTAG
GGAAGTTGTTTTTTGGGATGTTTAAATCGAGTTGTGTTTGGAGTGATGTTTAAGTTA
ATGTTAGGGTAAGGTAATAGTTTTTGGTCGTTTTTTAGTATTTTTGTAATGTATATG
AGTTCGGGAGATTAGTATTTAAAGTTGGAGGTTCGGGAGTTTAGGAGTTGGCGGAGG
GCGTTCGTTTTGGGATTGTATTTGTTTTCGTCGGGTCGTTCGGTTTTATCGGATTCG
TAGGTTTTCGGGGTAGGGTCGGGGTTAGAGTTCGCGTGTCGGCGGATATGCGTTGC
GTCGTTTTTAATTTCGGGTTGTGTTTTTTTTTAGGTGGTTCGTCGGTTTTTGAGTT
TTTTGTTTTG]CGGGGATACGGTTTGTATTTTGTTCGCGGTTACGGATTATGATTAT
GATTTTTTATATTAAAGTATTTGGGATGGTTTTATTGT

Figur 4: Analyse der Reamplifikation der ESR1 PCR-Produkte (vgl. Fig. 4) in einem 1,5 %igen Agarosegel (Manniatis et al.). Die Reamplifikation der PCR-Produkte der Primerkombinationen ER1-B-U/ER1-B-L (Spur2,7,12), ER1-B-L-M13b/ ER1-B-L-M13a (Spur3,8,13), ER1-B-U-M13a/ ER1-B-L-M13a (Spur 4,8,14) und ER1-B-L-M13b/ ER1-B-U-M13b (Spur 5,10,15) mit generischen Primern und Primerkombinationen wurde untersucht. Jeweils 4 µl der PCR-Reaktionen mit den generischen Primerkombinationen M13a/M13b (Spur 1-5), des generischen Primers M13a (Spur 6-10) und des generischen Primers M13b (sSpur 11-15) sind dargestellt. Spur M ist ein DNA-Fragment-Größenstandard.

### Kurzbeschreibung der Figuren

Figur 1: Genspezifische Amplifikation von OAT und MDR1.
   M Größenmarker,
   A, Amplifikation von OAT (Primer: OAT-f1mp und OAT-rmp),
   B, Amplifikation von OAT (Primer: OAT-f2mp und OAT-rmp),
   C, Amplifikation von MDR1 (Primer: MDR-f1mp und MDR-rmp) und
   D, Amplifikation von MDR1 (Primer: MDR-f2mp und MDR-rmp)
Figur 2: Reamplifikation von OAT/Mdr1-Fragmenten aus Beispiel 2 mit generischen Primer.
   M Größenmarker,
   A, MDR1 mit Primer gen1-fp5 und gen-rp;
   B, OAT mit Primer gen1-fp5 und gen-rp;
   C, MDR1 und OAT mit Primer gen1-fp5 und gen-rp;
   D, MDR1 und OAT mit Primer gen2-fp5 und gen-rp.
Figur 3: Jeweils 4 µl der PCR-Reaktionen der Primerkombinationen ER1-B-L-M13b/ ER1-B-L-M13a (Spur 1,5), ER1-B-UM13a/ ER1-B-L-M13a (Spur 2,6), ER1-B-L-M13b/ ER1-B-U-M13b (Spur 3,7) und ER1-B-U/ER1-B-L (Spur 4,8) auf Bisulphit behandelter DNA (Spur 1-4) und unbehandelter genomischer DNA (Spur 5-8) wurden analysiert. Spur M ist ein DNA-Fragment-Größenstandard.
Figur 4: Analyse der Reamplifikation der ESR1 PCR-Produkte (vgl. Fig. 4) in einem 1,5 %igen Agarosegel (Manniatis et al.).

### Anhang: Sequenzen von OAT und MDR1 nach der Behandlung mit Bisulfit:

Mdr1:

0 GTTAAGAATA AAATATATTA AGATAAGGAA AATTTGTAGT TAAGAATAGA AAAAAATTAT
60 GGTTTTGAAG TATGAGTTAT TTAAAGAAAG TGGAAATATT TTTAGATTAT GTAGTAAAAA
120 ATAAAGTGAT TTTTTTTTTT TAAATTTATG TAATAAATTG ATAGGTAATA TGTGAAAGTT
180 ATAGAATGTA GATTAGAGGA TATAATAAAT TTATTTTTTT TATGTTTATA AGAAGTAAGA
240 AAAGTTTTGA TGTGAGTTAG TATTGTTTTA TAATTTTGAA TTGTGTAGAT TGTACGTATT
300 TTTTTTTAGT TTGAAGTAAA TAGTGGATAG GAAAAAATAT TAAATGTTGG TAGTAAATAT
360 GGAAGGAAAT TATAATTAAT GTAATATGTT AAAATATGTT ATGTTTATTT TATTAATTTG
420 AATTAAAATG TAAGAATTTA AAATGTTTTG GAAAAATACG GGTATTGATT TGACGTTTGA
480 AGTTTTAAAA TATTATATAT TTTGAAATAG TATTTGTaTT TTGAAATATT TGTTTTTATA
540 TATTTTTTAA AATTTTTTTT TTTTTTTATT TTATTTATTA TTAAATAAAG GATGAATAGA
600 TGTAATTTAG AAATTGTTAA GTATGTTGAA GAAAGATTAT TGTAGAAAAA TTTTTTTTAG
660 TTTTTTTAAA GGTGTTAGGA AGTAGAAAGG TGATATAGAA TTGGAGAGGT CGGAGTTTTT
720 GTATTAATTG TATTAAATGC GAATTTCGAG AAAATTTTTT TTAATTACGT TTTGTAGTTA
780 TATGGATATG AAGATTTATG TGAATTTTGA AAGACGTGTT TATATAAGTT GAAATGTTTT
840 TAATGATTTA GTTGATGCGC GTTTTTTTAT TTGTTTTTTT TAGAGAGGTG TAACGGAAGT
900 TAGAATATTT TTTTTGGAAA TTTAATTTGT TTCGTAGTTT TTCGAGGAAT TAGTATTTAG
960 TTAATTCGGG TCGGGAGTAG TTATTTGTGG TGAGGTTGAT TGGTTGGGTA GGAATAGCGT
1020 CGGGGCGTGG GTTGAGTATA GTCGTTTCGT TTTTTTTGTT ATAGGAAGTT TGAGTTTATT
1080 CGAGTAGCGG TTTTTTTAAG TTTAAAGAAG TAGAGGTCGT TGTTCGTTTT TTTTAGGTTT
1140 TTTTATTAAA GTCGGAGTAT TTTTTTTTAA AATTTTACGT TTTGGTGGTC GTTTTAAGGA
1200 GCGCGAGGTA GGGGTACGTA AAGTTGGGAG TTATTATGGG ATAGTTTTTA AGTGTTAGGT
1260 TTTTAGATTT TTTGAATTTG GTTTTTACGG GAGAAGGGTT TTTTGAGGCG TGGATAGTGT
1320 GAAGTTTTTT GGTAAGTTTA TGGGGATTAA GTGGGGTTAG ATTTAGATTT AGGAGTTTTT
1380 GGAGTAGCGT TTAAATCGTA GTGGTATTGG ATTATGTTGT TCGGAGCGCG TATAGTTCGC

1440 GCGGTGCGGG GATTTGTTTT TTGAGTTCGC GGGCGGTGGG TGGGAGGAAG TATCGTTCGC
1500 GGCGATTGGA ATCGGGAGGG AGAATCGTAT TGGCGGCGGG TAAAGTTTAG AACGCGTTGT
1560 TAGATTTTTA ATTTTGTTTT CGTGGAGATG TTGGAGATTT CGCGTATAGG AAAGTTTTTG
1620 TAGTGTTTAT CGCGGTTAGA GTAGTTGGGG TATTAACGGC GGGCGTTTTT TTTTATTGTT
1680 TTTTGGTTTC GACGGGGGAT TAGAGGTTAG TTTTATTTTT AGCGCGTTTG AGGTTTATGT
1740 ATTTGGTTAA TGAGTTGCGG TTTTTTTTTA GGTCGGGATG GATTTTGAAG GGGATCGTAA
1800 TGGAGGAGTA AAGAAGAAGA ATTTTTTTAA ATTGAATAAT AAAAGGTAAT TAGTTTGTTT
1860 TATTTTTATA GTTTATATAG TTGCGAGATT TGAGTAATTT ATTTTTAGTT TTTAGTTTTG
1920 AAATAAATGA TATGTTGTTG TTTTTAATTA TTTTTAAGAA ACGTAAGTTA GTTTTTGGAA
1980 TTAATATTTT TGTTTAGAGT AGAAGTTTGT TGGTTGAGTG GAGTATAGTA TATGTATTTT
2040 TTTTGTTTTT TTTGTTTTTT TTTTTAATGA TATATAATAT TTTATATATT TATGAAATGG
2100 GGTATATGGA AGCGTTTTTT ATATGTTCGG AATGTGTAAT GATTAAGTTC GGGTATTTGA
2160 AGGATATATT ATTTTAGGTA TATTTTATTT TTATGTGTTG ATAATATTTT AAGTTTTTTA
2220 GTTATTTTGA AATATATAAT ATATTGTTAA TTGTAGTTAT TTTCGTTTGT TATCGAATAT
2280 TGGAATTTAT TTGTTTTATT TAATCGTTTT TAGTTATTTA TTAATTTTTT TTTATTTTAT
2340 TTTTTTATTT TTTTCGGTTT TTTTTTTTTA GTTTTGGTGT GTTTTTTTTT TAGTTTTTTT
2400 GTTTTAGATA GGCGGATGTT TATATGTGTT TTTGTTTTAT GAATTTTTGT TTTTTAAGTG
2460 GTGTTGGTCG TTTATACGTG AGTTATATGT TGTTGGTGAT TTGTTTTGTG GTTTAGGTTT
2520 TTGTTTTCGG TAAATGGTTA TGTAAATATC GCGTTTGTGG TTTGGTTGAT GAGATAGAAG
2580 GTTAAAAGTA TATTTAGGTT GTTAATTGGT AATAAATATT TGTATATAAT ATTGGTAATG
2640 TAATTATATA GGGAAAATAA TTATTTAAAG TAAATTTTGA TTATGGTGTT TTGTTTTTAT
2700 AGAATATTTA AAATTTTATT AAATAGATTT ATTGTTAGTA GTAAATTGTA AAATAGATTA
2760 GTAAGTTTAA TAATATTAGA AATTGTAATG TAAATTATAA GATAAATTAG TTAAATATAT
2820 TAATATTATA AGAAATTAAG TTTTTTAGTG TAAGAGAAAA AATATAAATG TGGAAATTAA
2880 ATATATTTTT AAAAATAATG TTAAGTTTGA ATTAGAAATT TTAATATGAA TT

OAT:

1 GGTAGCGACG ATTTTTGGAG GTGGATTTAG AGGTATAATT AAGTCGCGCG GCGTATTAGG
61 GTTTAAGGGT ATGGGGTTTT CGTAGTTGTG GTTGGGGTAG AGTTGGGGTT GTTTTTTTTT
121 TTAGGAGTAT AGGCGGCGGT TTAGTTTTAC GTTTTTCGTT TTTAGTTATA TTCGGTTCGC
181 GTAGTGGGGG GTTTAATAGA TTTTTTTTTT TCGGGTTTTA GTTTTTTCGT TAGTAAGGGC
241 GGATAAGGAT TTTTTTCGTT TCGTTAGAGG AGGCGATCGA GGGGTTTGAG TTTAGGTATA
301 GGTCGGCGGG TTTAGGAGGC GCGAGGCGGA TCGAATTCGC GGGAGGAGTA AAGATTTTTG
361 ATGCGCGGTC GGAGGGCGGG GCGGAGGACG GGATTTACGC GATTGGTATT TTGTTTTTCG
421 TTTTAGTTAA TGAGCGGCGA GGGTGTTTTG GGGGCGGGGT AGAATTAGTT TTTAAGTTGT
481 AGTGACGTTT CGGCGTTATT GTTGCGTTTT ATAGACGTCG CGTGTATTCG GTTGTTTTTA
541 GGCGTTGTTA GGTATCGTTT GGGCGTCGTT GTTTTGGGGT TTTGGTTCGG GTTTGGTCGG
601 AAACRTCACT ACAACTTAAA AACTAA

## Patentansprüche

1. Verfahren zur Amplifikation von Nukleinsäuren, **dadurch gekennzeichnet, dass** die folgenden Schritte ausgeführt werden:
a) eine Nukleinsäureprobe wird mit einem Reagenz chemisch umgesetzt, wobei 5-Methylcytosin unverändert bleibt und Cytosin in Uracil oder eine andere im Basenpaarungsverhalten dem Uracil ähnliche Base umgewandelt wird.
b) die zu amplifizierenden Abschnitte werden mit mindestens zwei Primeroligonukleotiden hybridisiert, welche zwei Domänen aufweisen, von denen die am 3'-Ende befindliche, sequenzspezifische Domäne an den zu amplifizierenden Abschnitt hybridisiert, während die am 5'-Ende befindliche, generische Domäne nicht hybridisiert,
c) es wird eine erste Amplifikationsreaktion mittels einer Polymerase durchgeführt,
d) an die Amplifikate wird ein markiertes Primeroligonukleotid hybridisiert, welches an die generische Domäne der ersten Primer hybridisiert und
e) das Amplifikat hinsichtlich seiner Sequenz untersucht, weiterhin **dadurch gekennzeichnet, dass** die generische Domäne der Primer die Nukleobasen A, C, G und T enthält, während die sequenzspezifische Domäne entweder nur die Basen A, T und C oder aber nur die Basen A, T und G enthält.

2. Verfahren nach Anspruch 1, **dadurch gekennzeichnet, dass** nach Schritt d des Anspruchs 1 zusätzlich eine zweite Amplifikation mit einer Polymerasereaktion erfolgt.

3. Verfahren nach einem der vorstehenden Ansprüche, **dadurch gekennzeichnet, dass** es sich bei dem Reagenz um ein Bisulfit (=Hydrogensulfit, Disulfit) handelt.

4. Verfahren nach einem der vorstehenden Ansprüche, **dadurch gekennzeichnet, dass** die chemische Behandlung nach Einbetten der DNA in Agarose erfolgt.

5. Verfahren nach einem der vorstehenden Ansprüche, **dadurch gekenntzeichnet, dass** bei der chemischen Behandlung ein die DNA-Duplex denaturierendes Reagenz und/oder ein Radikalfänger zugegen ist.

6. Verfahren nach einem der vorangehenden Ansprüche, **dadurch gekennzeichnet, dass** es sich bei den Markierungen der Oligonukleotidprimer um Fluoreszenzfarbstoffe mit unterschiedlichem Emissionsspektrum (z. B. Cy3, Cy5, FAM, HEX, TET oder ROX) oder um Fluoreszenzfarbstoff-Kombinationen im Falle von Energietransfer-Fluoreszenzfarbstoff markierten Primern handelt.

7. Verfahren nach einem der voranstehenden Ansprüche, **dadurch gekennzeichnet, dass** die Markierungen Radionuklide sind.

8. Verfahren nach einem der voranstehenden Ansprüche, **dadurch gekennzeichnet, dass** die Markierungen ablösbare Massenmarkierungen sind, die in einem Massenspektrometer nachgewiesen werden.

9. Verfahren nach einem der voranstehenden Ansprüche, **dadurch gekennzeichnet, dass** zur Markierung Moleküle verwendet werden, die erst in einer weiteren chemischen Reaktion ein Signal erzeugen.

10. Verfahren nach einem der voranstehenden Ansprüche, **dadurch gekennzeichnet, dass** zur Markierung Moleküle verwendet werden, die an definierten Stellen an einer Festphase immobilisiert werden.

11. Verfahren nach einem der voranstehenden Ansprüche, **dadurch gekennzeichnet, dass** die PCR-Fragmente auf einer Festphase in Form eines rechtwinkeligen oder hexagonalen Gitters angeordnet sind.

12. Verfahren nach einem der voranstehenden Ansprüche, **dadurch gekennzeichnet, dass** an den Amplifikaten angebrachte Markierungen an jeder Position der Festphase, an der sich eine Oligonukleotidsequenz befindet, identifizierbar sind.

13. Verfahren nach einem der voranstehenden Ansprüche, **dadurch gekennzeichnet, dass** für die Amplifikation eine hitzebeständige DNA-Polymerase verwendet wird.

14. Verfahren nach einem der voranstehenden Ansprüche, wobei die Nukleinsäure aus einer genomischen DNA-Probe erhalten wurde, wobei Quellen für DNA z. B. zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

15. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Batwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

16. Verwendung eines Verfahrens nach einem der Ansprüche 1 bis 15 zur Unterscheidung von Zelltypen oder Geweben oder zur Untersuchung der Zelldifferenzierung.

## Claims

1. A method for the amplification of nucleic acids, **characterized in that** the following steps are conducted:
a) a nucleic acid sample is chemically converted with a reagent, whereby 5-methylcytosine remains unchanged and cytosine is converted to uracil or another base similar to uracil in its base-pairing behavior,
b) the segments to be amplified are hybridized with at least two primer oligonucleotides, which have two domains, of which the sequence-specific domain found on the 3'-end hybridizes to the segment to be analyzed, while the generic domain found on the 5'-end does not hybridize,
c) a first amplification reaction is conducted by means of a polymerase,
d) a labeled primer oligonucleotide, which hybridizes to the generic domain of the first primer, hybridizes to the amplificate, and
e) the sequence of the amplificate is investigated, further **characterized in that** the generic domain of the primers contains the nucleobases A, C, G and T, while the sequence-specific domain contains either only the bases A, T and C or only the bases A, T and G.

2. The method according to claim 1, further **characterized in that** after step d) of claim 1, a second amplification with a polymerase chain reaction is additionally conducted.

3. The method according to one of the preceeding claims, further **characterized in that** the reagent involves a bisulfite (= hydrogen sulfite, disulfite).

4. The method according to one of the preceeding claims, further **characterized in that** the chemical treatment is conducted after embedding the DNA in agarose.

5. The method according one of the preceeding claims, further **characterized in that** in the chemical treatment, a reagent that denatures the DNA duplex and/or a radical trap are added.

6. The method according to one of the preceding claims, further **characterized in that** the labeling of oligonucleotide primers involves fluorescent dyes with different emission spectra (e.g., Cy3, Cy5, FAM, HEX, TET or ROX) or fluorescent dye combinations in the case of primers labeled by energy-transfer fluorescent dye.

7. The method according to one of the preceding claims, further **characterized in that** the labels are radionuclides.

8. The method according to one of the preceding claims, further **characterized in that** the labels are removable mass labels which are detected in a mass spectrometer.

9. The method according to one of the preceding claims, further **characterized in that** molecules that only produce a signal in a further chemical reaction are used for the label.

10. The method according to one of the preceding claims, further **characterized in that** molecules which are immobilized at defined positions on a solid phase are used for the label.

11. The method according to one of the preceding claims, further **characterized in that** the PCR fragments are arranged on a solid phase in the form of a rectangular or hexagonal grid.

12. The method according to one of the preceding claims, further **characterized in that** labels that are introduced on the amplificates at each position of the solid phase at which an oligonucleotide sequence is found can be identified.

13. The method according to one of the preceding claims, further **characterized in that** a heat-stable DNA polymerase is used for the amplification.

14. The method according to one of the preceding claims, wherein the nucleic acids were obtained from a genomic DNA sample, whereby sources for DNA include, e.g., cell lines, blood, sputum, stool, urine, cerebrospinal fluid, tissue embedded in paraffin, for example, tissue from eyes, intestine, kidney, brain, heart, prostate, lung, breast or liver, histological slides and all possible combinations thereof.

15. Use of a method according to one of the preceding claims for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical, psychological and social consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as a consequence of an abnormality in the development process; malfunction, damage or disorder of the skin, the muscles, the connective tissue or the bones; endocrine and metabolic malfunction, damage or disease; headaches or sexual malfunction.

16. Use of a method according to one of the claims 1 to 15 for distinguishing cell types or tissues or for investigating cell differentiation.

## Revendications

1. Procédé d'amplification d'acides nucléiques, **caractérisé en ce que** les étapes suivantes sont exécutées :
a) un échantillon d'acide nucléique est soumis à une réaction chimique avec un réactif, la 5-méthylcytosine demeurant intacte et la cytosine étant transformée en uracile ou une autre base similaire à l'uracile dans le comportement d'appariement des bases,
b) les segments à amplifier sont hybridés avec au moins deux oligonucléotides amorces qui présentent deux domaines parmi lesquels le domaine spécifique à une séquence se trouvant à l'extrémité 3' s'hybride au segment à amplifier tandis que le domaine générique se trouvant à l'extrémité 5' ne s'hybride pas,
c) une première réaction d'amplification est exécutée au moyen d'une polymérase,
d) un oligonucléotide amorce marqué qui s'hybride au domaine générique des premières amorces est hybridé aux amplificats et
e) l'amplificat est analysé quant à sa séquence,
**caractérisé, en outre, en ce que** le domaine générique des amorces contient les nucléobases A, C, G et T, tandis que le domaine spécifique à une séquence contient soit uniquement les bases A, T et C, soit uniquement les bases A, T et G.

2. Procédé selon la revendication 1, **caractérisé en ce qu'**une seconde amplification avec une réaction de polymérase est exécutée, en plus, après l'étape d de la revendication 1.

3. Procédé selon l'une des revendications précédentes, **caractérisé en ce qu'**il s'agit, concernant le réactif, d'un bisulfite (=hydrogénosulfite, disulfite).

4. Procédé selon l'une des revendications précédentes, **caractérisé en ce que** le traitement chimique est effectué après enrobage de l'ADN dans l'agarose.

5. Procédé selon l'une des revendications précédentes, **caractérisé en ce qu'**un réactif dénaturant le duplex d'ADN et/ou un piège à radicaux est présent lors du traitement chimique.

6. Procédé selon l'une des revendications précédentes, **caractérisé en ce qu'**il s'agit, concernant les marqueurs des amorces d'oligonucléotide, de colorants fluorescents présentant un spectre d'émission différent (par exemple Cy3, Cy5, FAM, HEX, TET ou ROX) ou de combinaisons de colorants fluorescents dans le cas d'amorces marquées par un colorant fluorescent à transfert d'énergie.

7. Procédé selon l'une des revendications précédentes, **caractérisé en ce que** les marqueurs sont des radionucléides.

8. Procédé selon l'une des revendications précédentes, **caractérisé en ce que** les marqueurs sont des marqueurs de masse amovibles qui sont détectés dans un spectromètre de masse.

9. Procédé selon l'une des revendications précédentes, **caractérisé en ce que** l'on utilise, pour le marquage, des molécules qui ne produisent un signal que dans une réaction chimique ultérieure.

10. Procédé selon l'une des revendications précédentes, **caractérisé en ce que** l'on utilise, pour le marquage, des molécules qui sont immobilisées à des endroits définis sur une phase solide.

11. Procédé selon l'une des revendications précédentes, **caractérisé en ce que** les fragments de RPC sont disposés sur une phase solide sous forme d'une grille rectangulaire ou hexagonale.

12. Procédé selon l'une des revendications précédentes, **caractérisé en ce que** les marqueurs fixés aux amplificats peuvent être identifiés au niveau de chaque position de la phase solide où se trouve une séquence d'oligonucléotide.

13. Procédé selon l'une des revendications précédentes, **caractérisé en ce que** l'on utilise, pour l'amplification, une ADN polymérase résistante à la chaleur.

14. Procédé selon l'une des revendications précédentes, l'acide nucléique ayant été obtenu à partir d'un échantillon d'ADN génomique, des sources d'ADN comprenant par exemple des lignées cellulaires, du sang, des crachats, des fèces, de l'urine, du liquide céphalo-rachidien, un tissu enrobé dans la paraffine, par exemple un tissu oculaire, intestinal, rénal, cérébral, cardiaque, prostatique, pulmonaire, mammaire ou hépatique, des porte-objets histologiques et toutes combinaisons possibles de ces sources.

15. Utilisation d'un procédé selon l'une des revendications précédentes pour le diagnostic et/ou pronostic d'événements préjudiciables pour des patients ou des individus, ces événements préjudiciables appartenant à au moins une des catégories suivantes : effets médicamenteux indésirables ; cancers ; dysfonctionnements, dommages ou maladie du SNC ; symptômes d'agression ou troubles du comportement ; conséquences cliniques, psychologiques et sociales de dommages cérébraux ; troubles psychotiques et troubles de la personnalité ; démence et/ou syndromes associés ; maladie, dysfonctionnement et dommage cardiovasculaire ; dysfonctionnement, dommage ou maladie du tractus gastro-intestinal ; dysfonctionnement, dommage ou maladie du système respiratoire ; blessure, inflammation, infection, immunité et/ou convalescence ; dysfonctionnement, dommage ou maladie du corps découlant d'une anomalie du processus de développement ; dysfonctionnement, dommage ou maladie de la peau, des muscles, du tissu conjonctif ou des os ; dysfonctionnement, dommage ou maladie endocrinien(ne) et métabolique ; maux de tête ou dysfonctionnement sexuel.

16. Utilisation d'un procédé selon l'une des revendications 1 à 15 pour la distinction de types de cellules ou de tissus ou pour l'étude de la différenciation cellulaire.
